# EUROPEAN PATENT APPLICATION

(11) **EP 3 643 340 A1**
(43) Date of publication of application: **29.04.2020**
(21) Application number: 18202409.1
(22) Date of filing: 24.10.2018
(51) Int. Cl.: A61M 1/28, A61M 1/16, A61J 1/20

(54) **APPARATUS, DISPOSABLE AND METHOD FOR POINT-OF-CARE ANALYSIS OF A PERITONEAL DIALYSIS EFFLUENT**

(71) Applicant: Fresenius Medical Care Deutschland GmbH, 61352 Bad Homburg (DE); Fresenius Medical Care AG & Co. KGaA, 61352 Bad Homburg (DE)
(72) Inventor: Chamney, Paul, Tring, Hertfordshire HP23 4EW (GB); Weiss, Stefan, 63152 Bad Homburg (DE); Staude, Birgit, 64319 Pfungstadt (DE)
(74) Representative: Herrmann, Uwe

(57) **Abstract**

The invention relates to an apparatus for or performing a peritoneal dialysis treatment on a patient, the apparatus comprising: a dialysis fluid inlet (20) for receiving fresh dialysis fluid from a reservoir (80); a patient terminal (30) for connecting a patient; a dialysis fluid outlet (40) for draining an effluent of used dialysis fluid; and an effluent container (50) comprising an effluent inlet and arranged to receive effluent from the dialysis fluid outlet (40) through the effluent inlet; wherein the effluent container further comprises an effluent outlet and an associated sample container (60) that is fluidly connected with the effluent outlet via a connector that comprises an openable closure (71). The invention further relates to a disposable for use in such apparatus, a set of such disposables and a method for analysing a sample from a peritoneal dialysis effluent.

## Description

The invention relates to an apparatus, a disposable and a method for point-of-care analysis of a peritoneal dialysis effluent.

In peritoneal dialysis treatment, samples from the effluent that is drained from a patient's peritoneum are periodically analysed to gather information that may be beneficial to safety, tolerability and success of the treatment.

For example, according to current guidelines, a so-called peritoneal equilibration test (PET) should be carried out at least twice a year. This test measures the transport of solutes across the peritoneal membrane of the patient and the results are taken as a basis for future prescriptions in terms of fill volume, osmotic concentration and the like. For such testing, several individual effluent samples need to be collected during the course of the treatment. For this purpose, effluent samples are channelled off from effluent directly flowing out from the peritoneal cavity in certain intervals.

Guidelines also suggest to regularly analyse effluent samples for urea levels to indicate the level of uremic toxin removal. These simple quality assurance tests are less burdensome than the PET tests, as the measured concentration should be representative of the average urea concentration over an entire cycle of a peritoneal dialysis treatment, which requires that the effluent from an entire cycle is collected and mixed in a drainage bag before sampling. The sample can hence simply be taken from the drainage bag after the cycle is completed. Such quality assurance test can be carried out monthly or at least quarterly.

The taking of samples from a drainage bag requires transferring part of the effluent from the drainage bag to a sample container. This can be done by removing, e.g. decanting or sucking out part of the effluent through the inlet opening once disconnected, or by destroying the drainage bag to access the effluent, e.g., by piercing with a needle or cutting.

The invention aims to provide means to simplify fluid sampling from a drainage bag.

Against this background, the invention relates to an apparatus for or performing a peritoneal dialysis treatment on a patient, the apparatus comprising: a dialysis fluid inlet for receiving fresh dialysis fluid from a reservoir; a patient terminal for connecting a patient; a dialysis fluid outlet for draining an effluent of used dialysis fluid; and an effluent container comprising an effluent inlet and arranged to receive effluent from the dialysis fluid outlet through the effluent inlet. According to the invention, the effluent container further comprises an effluent outlet and an associated sample container that is fluidly connected with the effluent outlet via a connector that comprises an openable closure.

During the peritoneal dialysis treatment the closure in the connector can be closed. After the entirety of the effluent of a cycle or of multiple cycles has been collected in the effluent container and has been allowed to mix or has been actively mixed by turning, shaking or the like, the closure is opened and a sample volume flows from the effluent container to the sample container, ideally by gravity. The connector is then resealed, for example by resealing the closure, and the sample container separated from the effluent container for analysis purposes.

The effluent container and/or the sample container may be configured as bags having flexible walls. The bags are preferably plastic bags.

In one embodiment, at least sections of the walls of the sample container are transparent or partly transparent This allows visual analysis and spectroscopic analysis such as absorption analysis in the visible wavelength region.

More specifically, the sample container may be a cuvette having flexible and transparent walls. Such cuvette could be positioned in a fixture which holds the cuvette in place and shape, such that at least a section of the cuvette adopts a shape of predefined thickness. The fixture may further comprise a slot to fix a camera device such as a smartphone in a predetermined position from the cuvette. This enables to carry out an instant spectroscopic analysis of the effluent, for example, at the patient's home only using a patient's smartphone. Certain clouding or colouring of the effluent could be an early warning indicator for beginning peritonitis or the like.

In an alternative embodiment, the sample container could also be a container having rigid walls.

In one embodiment, the sample container is pre-equipped with a colour reagent that is able to undergo a colour reaction with a constituent of the effluent. The colour reagent could be present in a solid or liquid form. For example, it could be filled in the inner volume of the sample container, coated on the inner walls of the sample container or incorporated to the matrix of the material forming for the sample container. The colour reagent could, for example, be such that it undergoes a colour reaction with urea, creatinine, glucose, proteins, leucocytes, erythrocytes, lactate or other constituents relevant for evaluation of dialysis safety and efficiency.

Further, the sample container may be pre-equipped with a colour reagent that is able to undergo a reaction with glucose or with glucose derivatives. This colour reagent may be a dilution volume or may be part of a dilution volume, such as a saline solution for the determination of glucose and/or glucose derivatives. The volume of the dilution is preferably 5 ml (± 1ml).

In one embodiment, the colour reagent may be contained in a separate compartment within the sample container. This way, the effluent may stay separate from the colour reagent until contact is actively established by a user, for example, by breaking a seal, such as a perforable membrane, between the main compartment of the sample container comprising the effluent and the separate compartment comprising the colour reagent. This way the contact between the effluent and the colour reagent is only established shortly before a spectroscopic analysis to obtain reliable data.

The connector is preferably a flexibly walled tube. The tube is preferably short and may be less than 10 cm or even less than 5 cm long.

The openable closure may be a clamp acting on the exterior of such tube. Alternatively, the openable closure may be a valve such as, for example, a stopcock valve.

The openable seal is preferably a seal that can reversibly be opened and closed. This way, the seal can be opened to allow a sample volume to flow from the effluent container to the sample container, and resealed prior detaching the sample contained for further analysis.

The connector may also comprise a frangible connection which is self-sealing upon separation. This allows for a very easy handling and also for using a seal that is a one-way open only.

The interior volume of the sample container is generally much smaller than the interior volume of the effluent container. For example, the interior volume of the sample container is between 1 and 10 ml, preferably between 3 and 7 ml.

The sample container may comprise an opening or line for degassing. This enables easier filling of the container by gravity. For example, the sample container can comprise a vent valve of an opening closed by an air permeable but fluid tight membrane.

In one embodiment, the sample container comprises an access opening for removing effluent from the sample container, wherein the access opening is preferably closed by a closure device. The closure device may be a device that can be reversibly opened and closed, such as a latch. The closure device may further be a one-way device such as a detachable foil. The access opening can be used to remove effluent for main or further analysis.

Some constituents of the effluent may need to be determined by analysis other than visual or spectroscopic or chemical analysis. Effluent may be removed from the sample container through the access opening for such further analysis.

The invention further relates to a disposable for use in an inventive apparatus, which comprises the effluent container, associated sample container and connector. In a preferred embodiment, the disposable further comprises the entire fluid system of the inventive apparatus, which connects the dialysis fluid inlet, the patient terminal and the dialysis fluid outlet. Preferred embodiments of the constituents of the disposable are described in connection with the inventive apparatus.

The disposable can be loaded on a machine prior any dialysis treatment for peritoneal dialysis to provide an inventive apparatus. After treatment, the disposable can be discharged.

The invention also relates to a set of disposables comprising at least one such disposable. The inventive disposable comprising the effluent container with associated sample container and connector could be supplied in conjunction with standard disposables where the effluent containers do not have associated sample containers. As such, packages of disposables which are suitable for periodic point-of-care sampling could be provided.

Still further, the invention relates to a method for analysing a sample from a peritoneal dialysis effluent, the method comprising: providing an inventive apparatus; detaching the sample container comprising an effluent sample from the effluent container; and carrying out a spectroscopic or chemical analysis of the effluent contained in the sample container.

The method may generally be carried out at a patient's home. The spectroscopic analysis may be carried out with standard electronic equipment available to the patient, which encompasses a camera and is suitable for data evaluation and transfer. For example, the electronic equipment may be a smartphone or tablet computer.

Detaching of the sample container can be carried out by cutting the connector, for example, the connector tube. Sterility is not critical. The connector may comprise two closure devices to be able to cut the connector between the devices and avoid draining from either of the effluent container and the sample container.

In case the sample container is a cuvette having flexible and transparent walls or partly transparent walls, the detached cuvette may, prior analysing, be positioned in a fixture to hold it in place and shape such that at least a section of the cuvette adopts a shape of predefined thickness. The spectroscopic analysis of the effluent should then be carried out to the effluent contained in the respective section of the cuvette. The fixture may further comprise a slot to fix a camera device such as a smartphone in a predetermined position from the cuvette. This enables to carry out an instant spectroscopic analysis of the effluent, for example, at the patient's home only using a patient's smartphone. Certain clouding or colouring of the effluent could be an early warning indicator for beginning peritonitis or the like.

In case the cuvette is pre-equipped with a colour reagent that is contained in a separate compartment within the sample container, the seal between the main compartment of the sample container comprising the effluent and the separate compartment comprising the colour reagent may be broken prior spectroscopic analysis. This way the contact between the effluent and the colour reagent is only established shortly before the spectroscopic analysis to obtain reliable data.

It is conceivable that the cuvette or any other sample container is or may be reused for a number of measurements, i.e. that the cuvette etc. is not a disposable which is discarded after a one use, although this embodiment is also subject to the present invention. After performing one measurement the measured effluent may be discarded, the cuvette etc. may be cleaned, such as flushed and is then ready for the next measurement.

In one embodiment the cuvette etc. may be pre-equipped with a number of compartments which preferably each contain the colour reagent which may be arranged in a separate compartment.

These features may an embodiment of the claimed apparatus and/or claimed disposable and/or claimed method.

Generally, when using the inventive apparatuses and methods, the effluent can be analysed for concentrations of urea, creatinine, glucose, proteins, leucocytes, erythrocytes, lactate or other constituents relevant for evaluation of dialysis safety and efficiency.

The inventive apparatus, disposable and method enable a patient to easily acquire and analyse an effluent sample of a peritoneal dialysis treatment in a home environment. Such point-of-care testing reduces or even eliminates the need to send samples to a hospital lab. The inventive apparatus, disposable and method are applicable to any peritoneal dialysis patient irrespective of whether they are treated by automated peritoneal dialysis (APD) or continuous ambulatory peritoneal dialysis (CAPD).

Further details and advantages of the invention will become apparent from the description of the following working example. The figures show:
- Figure 1:: a schematic illustration of an apparatus according to the invention; and
- Figure 2:: an enlarged view of the effluent container and sample container of such apparatus.

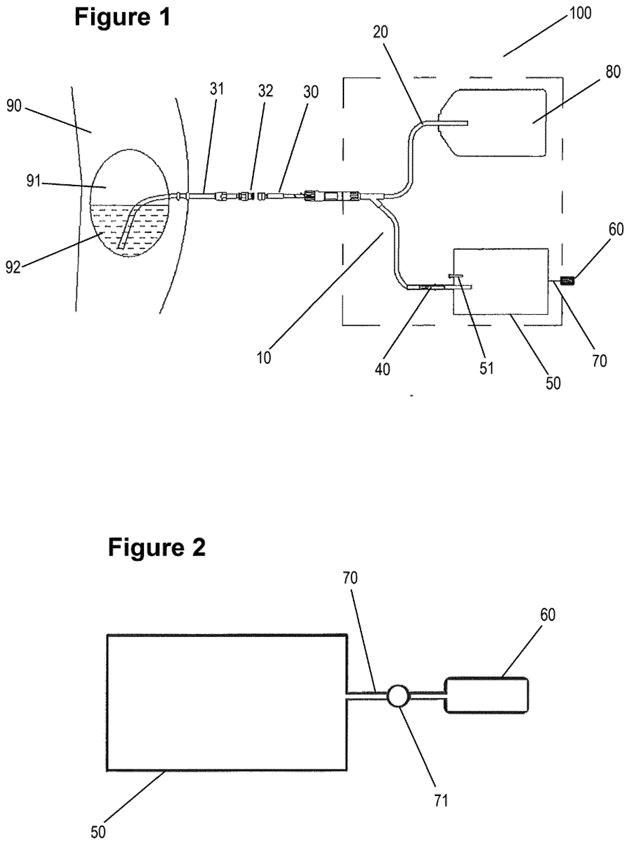

The apparatus 100 shown in Figure 1 is equipped with a disposable 10 that constitutes the fluid system of the apparatus 100.

Specifically, the disposable 10 comprises a dialysis fluid inlet 20 for receiving fresh dialysis fluid from a reservoir 80, a patient terminal 30 for connecting a patient 90, a dialysis fluid outlet 40 for draining an effluent of used dialysis fluid and a drain bag 50 to receive effluent from the dialysis fluid outlet 40. The drain bag 50 is equipped with a vent port 51 to facilitate the escape of air during filling.

The disposable 10 and/or the drain bag is loaded onto a peritoneal dialysis machine, also called cycler, to provide the inventive apparatus 100 for carrying out a peritoneal dialysis treatment on the patient 90. The reservoir 80 may be part of the disposable 10, just like the drain bag 50. The patient terminal 30 is linked to a catheter 31 by a Luer-connector 32. The catheter 31 extents to the peritoneal cavity 91 of the patient 90 to fill and drain peritoneal dialysis fluid 92 to the peritoneal cavity during a number of inflow-dwell-outflow cycles of the therapy.

Figure 2 shows an enlarged illustration of the drain bag 50. As apparent, the drain bag 50 comprises an associated sample bag 60, which is connected to the drain bag by a flexibly walled tube 70 and itself adopts the shape of a flexibly walled cuvette. All these items, i.e., the drain bag 50, the sample bag 60 and the flexibly walled tube 70 are formed from transparent polymer material. The flexibly walled tube 70 comprises a closure device 71 that may be a clamp, a valve or a self-sealing frangible. The sample bag 60 is formed as a cuvette. The sample bag 60 has a much smaller capacity than the main drain bag, e.g. only 5 ml.

During a peritoneal dialysis treatment, the closure device 71 in the tube 70 remains closed. Effluent from draining dialysis fluid 92 from the peritoneal cavity 91 of the patient 90 is collected and mixed in the drain bag 50. After the entirety of the effluent of a cycle or of multiple cycles has been collected in the effluent container and has been allowed to mix, the closure device 71 is opened and a sample volume flows from the drain bag 50 to the sample bag 60 through the tube 70 by gravity. The closure device 71 is then resealed and the tube 71 cut.

The detached sample bag 60 comprising an effluent sample is then mounted in a fixture to hold it in place and shape such that at least a section of the cuvette-shaped sample bag 60 adopts a shape of predefined thickness. A spectroscopic analysis of the effluent is then be carried out to the effluent contained in the respective section of the bag 60. For this purpose, the fixture further comprises a slot to fix a smartphone, which is able to record spectral data with its camera and is equipped with software to perform some basic analytic operations. The method may generally be a point-of-care analysis carried out at a patient's home.

The invention hence enables a simplified fluid sampling from the drain bag 50 by providing an apparatus and a disposable for peritoneal dialysis with bags comprising a main drain bag 50 as effluent container and a smaller sample bag 60 as sample container.

## Claims

1. An apparatus for or performing a peritoneal dialysis treatment on a patient, the apparatus comprising:
a dialysis fluid inlet for receiving fresh dialysis fluid from a reservoir;
a patient terminal for connecting a patient;
a dialysis fluid outlet for draining an effluent of used dialysis fluid; and
an effluent container comprising an effluent inlet and arranged to receive effluent from the dialysis fluid outlet through the effluent inlet;
**characterized in that**
the effluent container further comprises an effluent outlet and an associated sample container that is fluidly connected with the effluent outlet via a connector that comprises an openable closure.

2. The apparatus of claim 1, wherein the effluent container and/or the sample container are configured as bags having flexible walls.

3. The apparatus of any preceding claim, wherein at least sections of the walls of the sample container are transparent.

4. The apparatus of claims 2 and 3, wherein the sample container is a cuvette having rigid or partly rigid or flexible or partly flexible and transparent or partly transparent walls.

5. The apparatus of any preceding claim, wherein the sample container is pre-equipped with a colour reagent that is able to undergo a colour reaction with a constituent of the effluent.

6. The apparatus of any preceding claim, wherein the connector is a flexibly walled tube.

7. The apparatus of claim 6, wherein the openable closure is a clamp acting on the exterior of the tube.

8. The apparatus of any preceding claim, wherein the openable closure is a stopcock valve.

9. The apparatus of any preceding claim, wherein the openable seal is a seal that can reversibly be opened and closed.

10. The apparatus of any preceding claim, wherein the interior volume of the sample container is between 1 and 10 ml, preferably between 3 and 7 ml.

11. The apparatus of any preceding claim, wherein the sample container comprises an opening or line for degassing.

12. The apparatus of any preceding claim, wherein the sample container comprises an access opening for removing effluent from the sample container, wherein the access opening is preferably closed by a closure device.

13. A disposable for use in an apparatus of any preceding claim, the disposable comprising the effluent container, associated sample container and connector.

14. A set of disposables comprising at least one disposable of claim 13.

15. A method for analysing a sample from a peritoneal dialysis effluent, the method comprising:
providing an apparatus according to any one of claims 1-12;
detaching the sample container comprising an effluent sample from the effluent container; and
carrying out a spectroscopic or chemical analysis of the effluent contained in the sample container.

16. A method of claim 15, wherein the sample container is used for only one measurement or for a number of measurements, i.e. refillable with subsequent effluent samples.
